# EUROPEAN PATENT SPECIFICATION

(11) **EP 2 281 520 B1**
(45) Date of publication and mention of the grant of the patent: **02.10.2019**
(21) Application number: 10251399.1
(22) Date of filing: 05.08.2010
(51) Int. Cl.: A61B 17/34

(54) **Elongated seal anchor for use in surgical procedures**
Verlängerte, chirurgische Dichtungsanker
Ancrage d'étanchéité alongé pour une utilisation dans des procédures chirurgicales

(30) Priority: 06.08.2009 US 231781 P; 06.08.2009 US 231790 P; 06.08.2009 US 231798 P; 06.08.2009 US 231806 P; 11.06.2010 US 813861
(43) Date of publication of application: 09.02.2011
(73) Proprietor: Covidien LP, Mansfield, MA 02048 (US)
(72) Inventor: Kleyman, Gennady, Brooklyn, NY 11230 (US)
(74) Representative: Soames, Candida Jane

(56) References cited:
- EP-A1- 2 044 889
- WO-A1-01/49363
- WO-A1-2008/093313
- US-A- 5 375 588
- US-A- 5 842 971
- US-A1- 2005 096 695
- US-B1- 6 454 783

## Description

### BACKGROUND

### 1. Technical Field

The present disclosure relates to a seal for use in a surgical procedure. More particularly, the present disclosure relates to a seal anchor member adapted for insertion into an incision in tissue, and, for the sealed reception of one or more surgical objects such that a substantially fluid-tight seal is formed with both the tissue and the surgical object, or objects.

### 2. Background of the Related Art

Today, many surgical procedures are performed through small incisions in the skin, as compared to the larger incisions typically required in traditional procedures, in an effort to reduce both trauma to the patient and recovery time. Generally, such procedures are referred to as "endoscopic", unless performed on the patient's abdomen, in which case the procedure is referred to as "laparoscopic". Throughout the present disclosure, the term "minimally invasive" should be understood to encompass both endoscopic and laparoscopic procedures.

During a typical minimally invasive procedure, surgical objects, such as surgical access devices, e.g., trocar and cannula assemblies, or endoscopes, are inserted into the patient's body through the incision in tissue. In general, prior to the introduction of the surgical object into the patient's body, insufflation gasses are used to enlarge the area surrounding the target surgical site to create a larger, more accessible work area. Accordingly, the maintenance of a substantially fluid-tight seal is desirable so as to inhibit the escape of the insufflation gases and the deflation or collapse of the enlarged surgical site.

To this end, various valves and seals are used during the course of minimally invasive procedures and are widely known in the art. EP 2 044 889 A1 discloses a seal anchor member is adapted to transition from an expanded condition to a compressed condition so as to facilitate the insertion and securement thereof within a tissue tract in tissue.

A continuing need exists for a seal anchor member that can be inserted directly into an incision in tissue in a narrow area, such as a cavity between two ribs, and that can accommodate a variety of surgical objects while maintaining the integrity of an insufflated workspace.

### SUMMARY

The surgical apparatus according to the invention is defined in claim 1. Preferred embodiments are defined in the dependent claims.

A surgical apparatus for positioning within a tissue tract accessing an underlying body cavity includes a seal anchor member comprising a compressible material and being adapted to transition between a first expanded condition and a second compressed condition. The first expanded condition facilitates a securing of the seal anchor member within the tissue tract and in substantial sealed relation with tissue surfaces defining the tissue tract, and the second compressed condition facilitates an at least partial insertion of the seal anchor member within the tissue tract. The seal anchor member may be formed of a foam material, which may be at least partially constituted of a material selected from the group consisting of polyisoprene, urethane, and silicone. Alternatively, the seal anchor member may be formed of a gel material.

The seal anchor member includes proximal and distal ends that define oblong perimeters to facilitate the positioning of the seal anchor member within a tissue tract accessing an underlying body cavity. At least one of the proximal and distal ends of the seal anchor member exhibits an arcuate configuration, which may be either concave or convex.

The seal anchor member can be configured to a rolled state to fit nonlinearly into the tissue tract. The seal anchor member may also be cut to better suit a surgical procedure.

At least one port extends between the proximal and distal ends and is adapted for the reception of an object whereby compressible material defining the at least one port is adapted to deform to establish a substantial sealed relation with the object. The at least one port may contain at least an undercut to protect against fluid leaks. The seal anchor member may include a plurality of ports that may be configured linearly with respect to the major axis of the perimeter of at least one of the distal and proximal ends. Each port may be spaced equally from its neighboring ports.

### BRIEF DESCRIPTION OF THE DRAWINGS

Various embodiments of the present disclosure are described hereinbelow with references to the drawings, wherein:
**FIG. 1** is a top, perspective view of a surgical apparatus in accordance with the principles of the present disclosure shown in an expanded condition illustrating a seal anchor member positioned relative to the tissue;
**FIG. 2** is a side, schematic view of the seal anchor member of **FIG. 1**;
**FIG. 3** is a cross-sectional view of the seal anchor member of **FIG. 1** taken along section line **3-3** of **FIG. 1** illustrating a plurality of ports defining undercuts;
**FIG. 4** is a side, schematic view of a port of the seal anchor member of **FIG. 2** with a surgical object inserted therethrough;
**FIG. 5** is a perspective, schematic view of the seal anchor member of **FIG. 1** shown in a compressed condition prior to the insertion thereof into an incision in tissue;
**FIG. 6** is a perspective, schematic view of the seal anchor member of **FIG. 1** shown in the expanded condition and subsequent to its insertion into the incision;

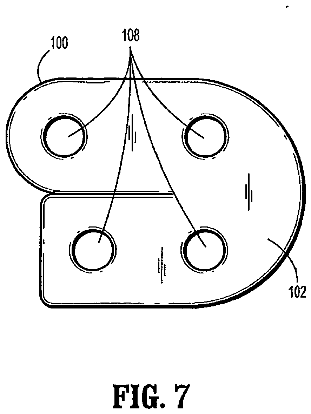

**FIG. 7** is a top, plan view of the seal anchor member of **FIG. 1** in a rolled state; and
**FIGS. 8A-8D** are perspective views of a surgical apparatus in accordance with another embodiment of the present disclosure illustrating a seal anchor member cut to varying lengths.

### DETAILED DESCRIPTION OF THE EMBODIMENTS

In the drawings and in the description which follows, in which like references numerals identify similar or identical elements, the term "proximal" will refer to the end of the apparatus which is closest to the clinician during use, while the term "distal" will refer to the end which is furthest from the clinician, as is traditional and known in the art.

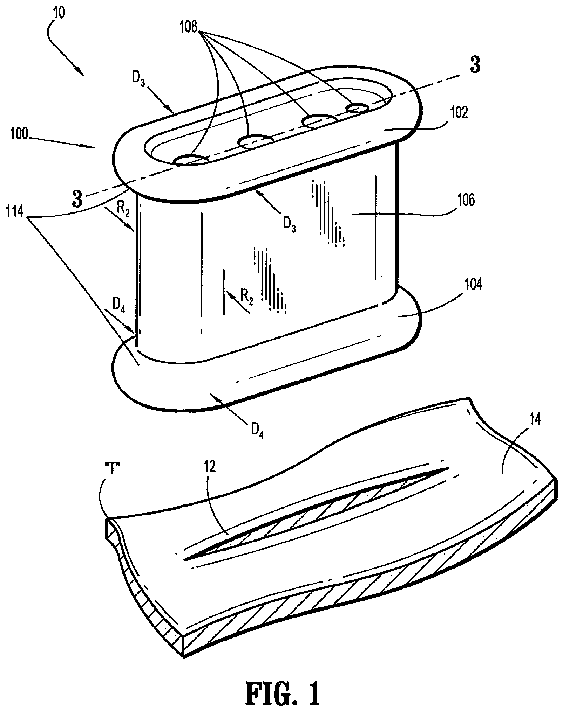

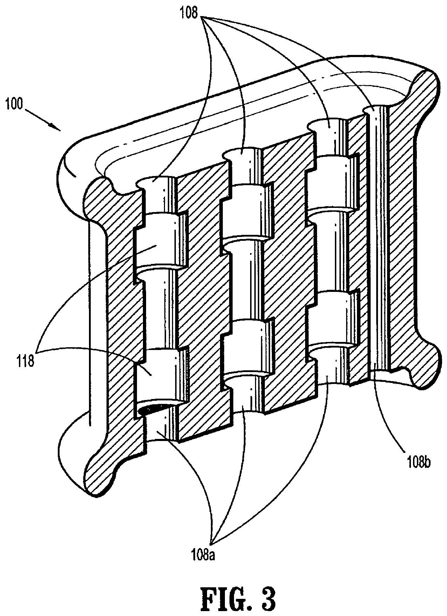

With reference to **FIGS. 1-4**, a surgical apparatus **10** for use in a surgical procedure, e.g., a minimally invasive procedure is illustrated. Surgical apparatus **10** includes seal anchor member **100** having proximal end **102** and distal end **104**. Seal anchor member **100** includes one or more ports **108** that extend through seal anchor member **100** between proximal end **102** and distal end **104**.

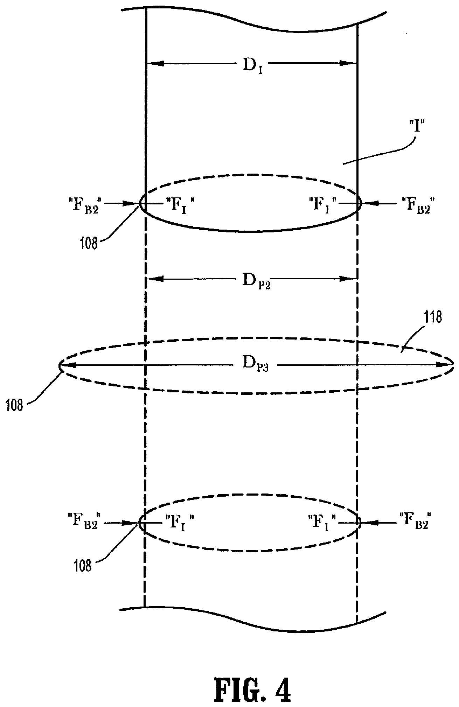

Seal anchor member **100** is formed from a suitable foam material having sufficient compliance to form a seal about one or more surgical objects, shown generally as surgical object "**I**" (**FIG. 4**), and also establish a sealing relation with tissue "**T**". The foam is sufficiently compliant to accommodate motion of the surgical object "**I**". In one embodiment, the foam includes a polyisoprene material.

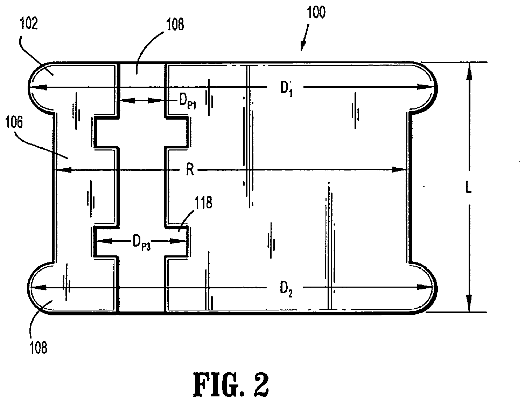

Proximal end **102** of seal anchor member **100** defines a first major axis **D₁** and distal end **104** defines a second major axis **D₂**. In one embodiment of seal anchor member **100**, the respective first and second major axes **D₁**, **D₂** of the proximal and distal ends **102**, **104** are substantially equivalent, as seen in **FIG. 2**, although an embodiment of seal anchor member **100** in which axes **D₁**, **D₂** are different is also within the scope of the present disclosure. As depicted in **FIG. 1**, positioning members **114** of proximal and distal ends **102**, **104** may define arcuate surfaces to assist in the insertion of seal anchor member **100** within a tissue tract **12** defined by tissue surfaces **14** and formed in tissue "**T**", e.g., an incision, as discussed in further detail below. Alternatively, proximal and distal ends **102**, **104** may define substantially planar surfaces or substantially arcuate surfaces. Embodiments are contemplated herein in which either or both of proximal and distal ends **102**, **104** define surfaces that are either or both arcuate or planar. The arcuate surfaces may be either or both concave or convex.

Intermediate portion **106** extends between proximal and distal ends **102**, **104** to define a dimension, or length, "**L**" therealong. Intermediate portion **106** further defines a dimension "**R**" substantially parallel to major axes **D₁**, **D₂**. The dimension "**R**" of intermediate portion **106** may remain substantially uniform along the dimension "**L**" thereof. Alternatively, the dimension "**R**" of intermediate portion **106** may vary along the dimension, or length, "**L**" thereof, thereby defining a cross-sectional dimension that varies along its length "**L**", which facilitates the anchoring of seal anchor member **100** within tissue "**T**".

The dimension "**R**" of intermediate portion **106** is appreciably less than the respective major axes **D₁**, **D₂** of proximal and distal ends **102, 104** to assist in anchoring seal anchor member **100** within tissue "**T**", as discussed in further detail below. However, in an alternate embodiment, the dimension "**R**" of intermediate portion **106** may be substantially equivalent to the respective major axes **D₁**, **D₂** of proximal and distal ends **102**, **104**. In cross section, intermediate portion **106** may exhibit any suitable elongated configuration, e.g., substantially oval or oblong, for insertion into a narrow incision.

Each port **108** is configured to removably receive the surgical object "**I**". Prior to the insertion of surgical object "**I**", port **108** is in a first state in which port **108** defines a first or initial dimension **D_{P1}**. Port **108** may define an opening within seal anchor member **100** having an initial open state. Alternatively, **D_{P1}** may be about **0**mm such that the escape of insufflation gas (not shown) through port **108** of seal anchor member **100** in the absence of surgical object "**I**" is substantially inhibited. For example, port **108** may be a slit extending the length "**L**" of seal anchor member **100** through proximal and distal ends **102**, **104**.

Upon the introduction of surgical object "**I**", port **108** transitions to a second state in which port **108** defines a second, larger dimension **D_{P2}** that substantially approximates the diameter **D_{I}** of surgical object "**I**" such that a substantially fluid-tight seal is formed therewith, thereby substantially inhibiting the escape of insufflation gas (not shown) through port **108** of seal anchor member **100** in the presence of surgical object "**I**". **D_{I}**, and thus **D_{P2}**, will generally lie within the range of about **5**mm to about **12**mm, as these dimensions are typical of the surgical objects used during the course of minimally invasive procedures. However, a seal anchor member **100** including a port **108** that is capable of exhibiting substantially larger, or smaller, dimensions in the second state thereof is not beyond the scope of the present disclosure. Seal anchor member **100** may include a plurality of generally tubular port segments (not shown) defining ports **108**. In addition, seal anchor **100** may be devoid of ports **108**. With this arrangement, ports **108** are created within seal anchor member **100** during the insertion of the surgical object "**I**". In accordance with this embodiment, seal anchor member **100** is formed of a flowable or sufficiently compliable material such as a foam material, e.g., an open-cell polyurethane foam, or a gel.

Ports **108** may include ports **108a**, which contain at least one undercut **118** that collects insufflation gas that leaks through the substantially fluid-tight seal between a surgical instrument "**I**" and a port **108a**. Each undercut **118** defines a diameter **D_{P3}** greater than **D_{P2}** and a length along a port **108a** less than "**L**". Insufflation gas that leaks through a substantially fluid-tight seal between an instrument "**I**" and a port **108a** may collect in an undercut **118** to inhibit further leakage of the gas through the substantially fluid-tight seal. Ports **108** may also include ports **108b,** which do not contain undercuts **118**, or any combination of ports **108a** and ports **108b.**

Generally, ports **108** are arranged linearly with respect to major axis **D₁**. Ports **108** may alternatively be arranged linearly with respect to major axis **D₂** or dimension "**R**". However, embodiments in which ports **108** are arranged nonlinearly, e.g., an oval or zigzag pattern, are also within the scope of this disclosure. Each port **108** may be spaced equally from its neighboring ports. However, embodiments in which ports **108** are spaced unequally are also within the scope of this disclosure.

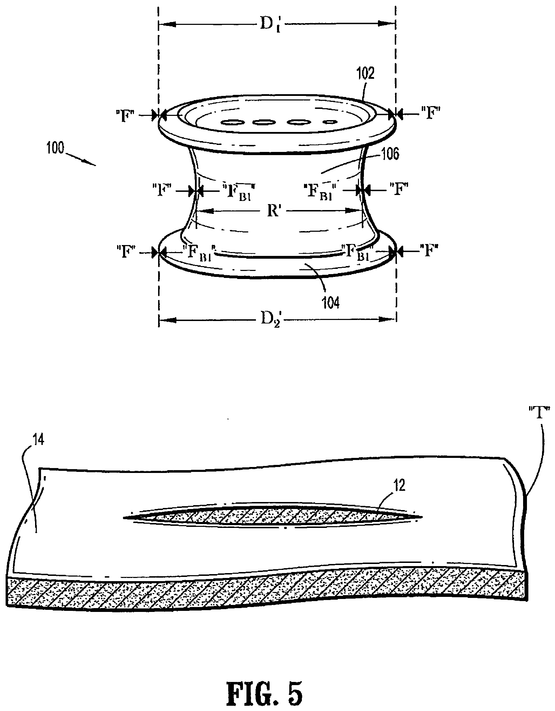

Referring now to **FIGS. 1** and **5**, seal anchor member **100** is adapted to transition from an expanded condition (**FIG. 1**) to a compressed condition (**FIG. 5**) so as to facilitate the insertion and securement thereof within tissue tract **12** in tissue "**T**". In the expanded condition, seal anchor member **100** is at rest and the respective major axes **D₁**, **D₂** of the proximal and distal ends **102, 104** of seal anchor member **100**, as well as the dimension "**R**" of the intermediate portion **106** are such that the seal anchor member **100** cannot be inserted within tissue tract **12**. However, as seen in **FIG. 5**, in the compressed condition, proximal and distal ends **102, 104** of seal anchor member **100** as well as intermediate portion **106** are dimensioned for insertion into tissue tract **12**.

Seal anchor member **100** is formed of a biocompatible compressible material that facilitates the resilient, reciprocal transitioning of seal anchor member **100** between the expanded and compressed conditions thereof. In one embodiment, the compressible material is a "memory" foam. An external force "**F**" is applied to seal anchor member **100** to cause the seal anchor member **100** to assume the compressed condition. External force "**F**" is directed inwardly and when seal anchor member **100** is subjected thereto, e.g., when seal anchor member **100** is squeezed, seal anchor member **100** undergoes an appreciable measure of deformation, thereby transitioning into the compressed condition.

As depicted in **FIG. 5**, as seal anchor member **100** is compressed under the influence of external force "**F**", an internal biasing force "**F_{B1}**" is created within seal anchor member **100** that is directed outwardly, opposing force "**F**". Internal biasing force "**F_{B1}**" endeavors to expand seal anchor member **100** and thereby return seal anchor member **100** to the expanded condition thereof. Accordingly, as long as seal anchor member **100** is subject to external force "**F**" greater than biasing force "**F_{B1}**", seal anchor member **100** is compressed, and as long as external force "**F**" equals biasing force "**F_{B1}**", seal anchor member **100** remains in the compressed condition. Upon the removal of external force "**F**", biasing force "**F_{B1}**" acts to return seal anchor member **100** to the expanded condition.

The compressible material comprising seal anchor member **100** also facilitates the resilient transitioning of port **108** between its first state (**FIGS. 1-3**) and its second state (**FIG. 5**). As previously discussed, prior to the insertion of surgical object "**I**", port **108** is in its first state in which port **108** defines a first or initial dimension **D_{P1}**. Port **108** may incorporate a slit extending the length "**L**" of seal anchor member **100**. In this first state, port **108** is at rest and is not subject to any external forces. However, upon the introduction of surgical object "**I**" through port **108** as depicted in **FIG. 4**, the surgical object "**I**" exerts a force "**F_{I}**" upon port **108** that is directed radially outward. Force "**F_{I}**" acts to enlarge the dimensions of port **108** and thereby transition port **108** into the second state thereof in which port **108** defines a second, larger dimension **D_{P2}** that substantially approximates the diameter **D_{I}** of surgical object "**I**". Consequently, an internal biasing force "**F_{B2}**" is created that is directed radially inward, in opposition to force "**F_{I}**". Internal biasing force "**F_{B2}**" endeavors to return port **108** to reduce the internal dimension of port **108** and thereby return port **108** to the first state thereof. Internal biasing force "**F_{B2}**" is exerted upon surgical object "**I**" and acts to create a substantially fluid-tight seal therewith. The significance of forces "**F_{B1}**" and "**F_{B2}**" will be discussed in further detail below.

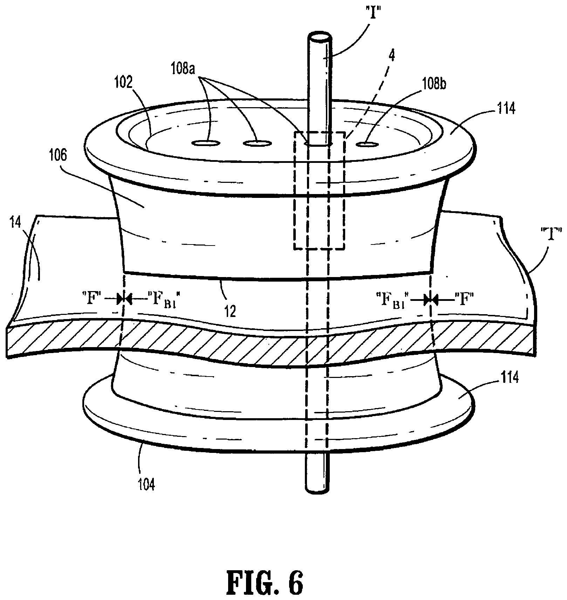

Referring again to **FIG. 1**, one or more positioning members **114** may be associated with either or both of proximal end **102** and distal end **104** of seal anchor member **100**. Positioning members **114** may be composed of any suitable biocompatible material that is at least semi-resilient such that positioning members **114** may be resiliently deformed and may exhibit any suitable elongated configuration, e.g., substantially oblong or oval. Prior to the insertion of seal anchor member **100**, positioning members **114** are deformed in conjunction with the respective proximal and distal ends **102**, **104** of seal anchor member **100** to facilitate the advancement thereof through tissue tract **12** (**FIG. 6**). Subsequent to the insertion of seal anchor member **100** within tissue tract **12**, the resilient nature of positioning members **114** allows positioning members to return to their normal, e.g., substantially oblong or oval, configuration, thereby aiding in the expansion of either or both of the respective proximal and distal ends **102**, **104** and facilitating the transition of seal anchor member **100** from its compressed condition to its expanded condition. Positioning members **114** also may engage the walls defining the body cavity to further facilitate securement of seal anchor member **100** within the body tissue. For example, positioning member **114** at leading end **104** may engage the internal peritoneal wall and positioning member **114** adjacent trailing end **102** may engage the outer epidermal tissue adjacent the incision **12** within tissue "**T**". In another embodiment of seal anchor member **100**, one or more additional positioning members **114** may be associated with intermediate portion **106**.

The use of seal anchor member **100** will be discussed during the course of a typical minimally invasive procedure. Initially, the peritoneal cavity (not shown) is insufflated with a suitable biocompatible gas, such as CO₂ gas, such that the cavity wall is raised and lifted away from the internal organs and tissue housed therein, providing greater access thereto. The insufflation may be performed with an insufflation needle or similar device, as is conventional in the art. Either prior or subsequent to insufflation, a tissue tract **12** is created in tissue "**T**", the dimensions of which may be varied dependent upon the nature of the procedure.

Prior to the insertion of seal anchor member **100** within tissue tract **12**, seal anchor member **100** is in its expanded condition in which the dimensions thereof prohibit the insertion of seal anchor member **100** into tissue tract **12**. To facilitate insertion, the clinician transitions seal anchor member **100** into the compressed condition by applying a force "**F**" thereto, e.g., by squeezing seal anchor member **100**. Force "**F**" acts to reduce the dimensions **D₁** and **D₂** of the proximal and distal ends **102**, **104**, respectively, to **D₁'** and **D₂'** (**FIG. 5**) including positioning members **114** (if provided) and to reduce the dimension "**R**" of intermediate portion **106** to "**R'**" such that seal anchor member **100** may be inserted into tissue tract **12**. As best depicted in **FIG. 6**, subsequent to its insertion, distal end **104**, positioning member **114** (if provided), and at least a section **112** of intermediate portion **106** are disposed beneath the tissue "**T**". Seal anchor member **100** is caused to transition from the compressed condition to the expanded condition by removing force "**F**" therefrom.

During the transition from the compressed condition to the expanded condition, the dimensions of seal anchor member **100**, i.e., the respective dimensions **D₁'**, **D₂'** (**FIG. 5**) of the proximal and distal ends **102**, **104** are increased to **D₁** and **D₂** (**FIG. 6**) and the dimension "**R'**" is increased to "**R**". The expansion of distal end **104** is relatively uninhibited given the disposition thereof beneath tissue "**T**", and accordingly, distal end **104** is permitted to expand substantially, if not completely. However, as seen in **FIG. 5**, the expansion of the section **112** of the intermediate portion **106** is limited by the tissue surfaces **14** (**FIG. 1**) defining tissue tract **12**, thereby subjecting intermediate portion **106** to an external force "**F**" that is directed inwardly. As discussed above, this creates an internal biasing force "**F_{B1}**" that is directed outwardly and exerted upon tissue surfaces **14**, thereby creating a substantially fluid-tight seal between the seal anchor member **100** and tissue surfaces **14** and substantially inhibiting the escape of insufflation gas around seal anchor member **100** and through tissue tract **12**.

In the expanded condition, the respective dimensions **D₁**, **D₂** of the proximal and distal ends **102**, **104** are larger than the dimension "**R**" of the intermediate portion **106**. Subsequent to insertion, the dimension **D₂** of distal end **104** and positioning member **114** is also substantially larger than the dimensions of the tissue tract **12**. Consequently, seal anchor member **100** may not be removed from tissue tract **12** in the expanded condition and thus, seal anchor member **100** will remain anchored within the tissue "**T**" until it is returned to its compressed condition.

After successfully anchoring seal anchor member **100** within the patient's tissue "**T**", one or more surgical objects "**I**" may be inserted through ports **108**. **FIG. 6** illustrates a surgical object "**I**" introduced through one of ports **108**. As previously discussed, prior to the insertion of surgical object "**I**", port **108** is in its first state in which port **108** defines an initial dimension **D_{P1}** which may be negligible in that port **108**, in one embodiment, is a slit. Accordingly, prior to the escape of insufflation gas through port **108**, in the absence of surgical object "**I**" is minimal, thereby preserving the integrity of the insufflated workspace.

Surgical object "**I**" may be any suitable surgical instrument and, accordingly, may vary in size. Suitable surgical objects to be introduced within one or more of the ports **108** include minimally invasive grasper instruments, forceps, clip-appliers, staplers, cannula assemblies, etc. Upon the introduction of surgical object "**I**", port **108** is enlarged, thereby transitioning into its second state in which port **108** defines a second dimension **D_{P2}** (**FIG. 4**) that substantially approximates the diameter **D_{I}** of surgical object "**I**", thereby creating a substantially fluid-tight seal with surgical object "**I**" and substantially inhibiting the escape of insufflation gas (not shown) through port **108** of seal anchor member **100** in the presence of a surgical object "**I**", as previously discussed.

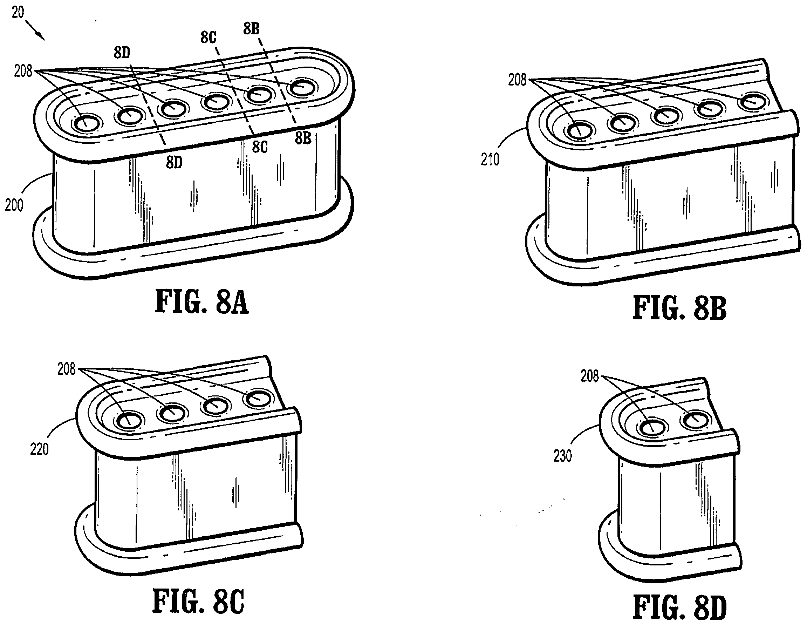

Turning now to **FIGS. 8A-8D**, a surgical apparatus, in accordance with an alternate embodiment of the present disclosure, is generally designated as **20**. Surgical apparatus **20** is substantially identical to surgical apparatus **10** and thus will only be discussed in detail herein to the extent necessary to identify differences in construction and operation thereof.

As seen in **FIG. 8A**, surgical apparatus **20** comprises a seal anchor member **200** defining a plurality of ports **208**. If seal anchor member **200** defines more ports **208** than are required for a particular surgical procedure, seal anchor member **200** may be cut to have a fewer number of ports **208**. **FIGS. 8B-8D** illustrate resulting seal anchor members **210**, **220**, and **230** when seal anchor member **200** is cut along segment lines **8B-8B**, **8C-8C**, and **8D-8D** respectively. Seal anchor member **200** and resulting seal anchor members **210**, **220**, and **230**, may be used in a surgical procedure in a substantially similar manner to seal anchor member 100 as discussed hereinbefore.

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope of the disclosure. The scope of the invention is defined by the claims.

## Claims

1. A surgical apparatus (10, 20) for positioning within a tissue tract (12) accessing an underlying body cavity, which comprises:
a seal anchor member (100, 200), the seal anchor member (100, 200) having proximal and distal ends (102, 104) and defining at least one port (108, 208) extending between the proximal and distal ends (102, 104) for reception of an object (I);
the seal anchor member comprising a compressible material such that:
the seal anchor member (100, 200) is adapted to transition between a first expanded condition to facilitate a securing of the seal anchor member (100, 200) within the tissue tract (12) and in substantial sealed relation with tissue surfaces defining the tissue tract (12), and a second compressed condition to facilitate an at least partial insertion of the seal anchor member (100, 200) within the tissue tract (12); and
the at least one port (108, 208) is adapted to deform to establish a substantial sealed relation with the object (I);
and wherein at least one of the proximal and distal ends (102, 104) exhibits an arcuate configuration to facilitate an insertion of the seal anchor member (100, 200) within the tissue tract (12);
**characterized in that** in the expanded condition the perimeters of the proximal and distal ends (102, 104) are oblong such that the seal anchor member is configured to a rolled state to facilitate the positioning within a tissue tract (12) accessing a narrow underlying body cavity.

2. The surgical apparatus (10, 20) according to claim 1, wherein each of the proximal and distal ends (102, 104) exhibits an arcuate configuration.

3. The surgical apparatus (10, 20) according to claim 2, wherein the arcuate configuration is concave, or the surgical apparatus (10, 20) according to claim 2, wherein the arcuate configuration is convex.

4. The surgical apparatus (10, 20) according to any preceding claim, wherein the seal anchor member (100, 200) is formed of a foam material.

5. The surgical apparatus (10, 20) according to claim 4, wherein the foam material is at least partially constituted of a material selected from the group consisting of polyisoprene, urethane, and silicone.

6. The surgical apparatus (10, 20) according to any of claims 1 to 3, wherein the seal anchor member (100, 200) is formed of a gel material.

7. The surgical apparatus (10, 20) according to any preceding claim, wherein the at least one port (108, 208) includes at least an undercut (118) to protect against leaks.

8. The surgical apparatus (10, 20) according to any preceding claim, wherein the surgical apparatus (10, 20) may be cut.

9. The surgical apparatus (10, 20) according to any preceding claim, wherein the seal anchor member (100, 200) includes a plurality of ports (108, 208).

10. The surgical apparatus (10, 20) according to claim 9, wherein the plurality of ports (108, 208) is configured linearly with respect to the major axis of the perimeter of at least one of the distal and proximal ends.

11. The surgical apparatus (10, 20)) according to claim 9, wherein each port (108, 208) of the plurality of ports (108, 208) is spaced equally from its neighboring ports.

12. The surgical apparatus (10, 20) according to claim 9, wherein the seal anchor member (100, 200) includes a plurality of generally tubular port segments, the port segments defining respective ports.

## Patentansprüche

1. Chirurgische Vorrichtung (10, 20) zum Positionieren innerhalb eines Gewebetraktes (12), der auf eine darunterliegende Körperhöhle zugreift, umfassend:
ein Dichtungsankerelement (100, 200), wobei das Dichtungsankerelement (100, 200) proximale und distale Enden (102, 104) aufweist und mindestens einen Anschluss (108, 208) definiert, der sich zwischen den proximalen und distalen Enden (102, 104) zur Aufnahme eines Objekts (I) erstreckt;
wobei das Dichtungsankerelement ein kompressibles Material umfasst, so dass:
das Dichtungsankerelement (100, 200) an den Übergang zwischen einem ersten aufgeweiteten Zustand zum Erleichtern einer Befestigung des Dichtungsankerelements (100, 200) innerhalb des Gewebetrakts (12) und in einer im Wesentlichen abgedichteten Beziehung zu Gewebeoberflächen, die den Gewebetrakt (12) definieren, und einem zweiten komprimierten Zustand angepasst ist, um eine zumindest teilweise Einführung des Dichtungsankerelements (100, 200) in den Gewebetrakt (12) zu erleichtern; und
der mindestens eine Anschluss (108, 208) zum Verformen angepasst ist, um eine im Wesentlichen abgedichtete Beziehung mit dem Objekt (I) herzustellen;
und wobei mindestens eines der proximalen und distalen Enden (102, 104) eine bogenförmige Konfiguration aufweist, um ein Einführen des Dichtungsankerelements (100, 200) in den Gewebekanal (12) zu erleichtern;
**dadurch gekennzeichnet, dass** im aufgeweiteten Zustand die Perimeter des proximalen und distalen Endes (102, 104) länglich sind, so dass das Dichtungsankerelement in einen gerollten Zustand konfiguriert ist, um die Positionierung innerhalb eines Gewebetraktes (12) zu erleichtern, der auf einen schmalen darunter liegenden Körperhöhle zugreift.

2. Chirurgische Vorrichtung (10, 20) nach Anspruch 1, wobei jedes der proximalen und distalen Enden (102, 104) eine bogenförmige Konfiguration aufweist.

3. Chirurgische Vorrichtung (10, 20) nach Anspruch 2, wobei die bogenförmige Konfiguration konkav ist, oder die chirurgische Vorrichtung (10, 20) nach Anspruch 2, wobei die bogenförmige Konfiguration konvex ist.

4. Chirurgische Vorrichtung (10, 20) nach einem der vorstehenden Ansprüche, wobei das Dichtungsankerelement (100, 200) aus einem Schaumstoff gebildet ist.

5. Chirurgische Vorrichtung (10, 20) nach Anspruch 4, wobei das Schaummaterial zumindest teilweise aus einem Material besteht, das ausgewählt ist aus der Gruppe bestehend aus Polyisopren, Urethan und Silikon.

6. Chirurgische Vorrichtung (10, 20) nach einem der Ansprüche 1 bis 3, wobei das Dichtungsankerelement (100, 200) aus einem Gelmaterial gebildet ist.

7. Chirurgische Vorrichtung (10, 20) nach einem der vorstehenden Ansprüche, wobei der mindestens eine Anschluss (108, 208) mindestens einen Hinterschnitt (118) zum Schutz vor Lecks aufweist.

8. Chirurgische Vorrichtung (10, 20) nach einem der vorstehenden Ansprüche, wobei die chirurgische Vorrichtung (10, 20) geschnitten werden kann.

9. Chirurgische Vorrichtung (10, 20) nach einem der vorstehenden Ansprüche, wobei das Dichtungsankerelement (100, 200) eine Vielzahl von Anschlüssen (108, 208) beinhaltet.

10. Chirurgische Vorrichtung (10, 20) nach Anspruch 9, wobei die Vielzahl von Anschlüssen (108, 208) linear in Bezug auf die Hauptachse des Umfangs von mindestens einem der distalen und proximalen Enden konfiguriert ist.

11. Chirurgische Vorrichtung (10, 20) nach Anspruch 9, wobei jeder Anschluss (108, 208) der Vielzahl von Anschlüssen (108, 208) gleichmäßig von ihren benachbarten Anschlüssen beabstandet ist.

12. Chirurgische Vorrichtung (10, 20) nach Anspruch 9, wobei das Dichtungsankerelement (100, 200) eine Vielzahl von im Allgemeinen röhrenförmigen Anschluss-Segmenten beinhaltet, wobei die Anschluss-Segmente entsprechende Anschlüsse definieren.

## Revendications

1. Appareil chirurgical (10, 20) pour positionner dans un tractus tissulaire (12) accédant à une cavité corporelle sous-jacente, qui comprend :
un élément d'ancrage étanche (100, 200), l'élément d'ancrage étanche (100, 200) ayant des extrémités proximale et distale (102, 104) et définissant au moins un orifice (108, 208) s'étendant entre les extrémités proximale et distale (102, 104) pour la réception d'un objet (I) ;
l'élément d'ancrage étanche comprenant un matériau compressible tel que :
l'élément d'ancrage étanche (100, 200) soit à même de passer entre un premier état expansé pour faciliter une fixation de l'élément d'ancrage étanche (100, 200) dans le tractus tissulaire (12) et en relation d'étanchéité sensible avec les surfaces définissant le tractus tissulaire (12), et un second état comprimé pour faciliter une insertion au moins partielle de l'élément d'ancrage étanche (100, 200) dans le tractus tissulaire (12) ; et
que le au moins un orifice (108, 208) soit à même de se déformer pour établir une relation étanche sensible avec l'objet (I) ; et
dans lequel au moins l'une des extrémités proximale et distale (102, 104) présente une configuration arquée pour faciliter une insertion de l'élément d'ancrage étanche (100, 200) dans le tractus tissulaire (12) ;
**caractérisé en ce que**, dans l'état expansé, les périmètres des extrémités proximale et distale (102, 104) sont oblongues de sorte que l'élément d'ancrage étanche soit configuré dans un état enroulé pour faciliter le positionnement dans un tractus tissulaire (12) accédant à une cavité corporelle sous-jacente étroite.

2. Appareil chirurgical (10, 20) selon la revendication 1, dans lequel chacune des extrémités proximale et distale (102, 104) présente une configuration arquée.

3. Appareil chirurgical (10, 20) selon la revendication 2, dans lequel la configuration arquée est concave ou appareil chirurgical (10, 20) selon la revendication 2, dans lequel la configuration arquée est convexe.

4. Appareil chirurgical (10, 20) selon l'une quelconque des revendications précédentes, dans lequel l'élément d'ancrage étanche (100, 200) est formé d'un matériau alvéolaire.

5. Appareil chirurgical (10, 20) selon la revendication 4, dans lequel le matériau alvéolaire est au moins en partie constitué d'un matériau choisi dans le groupe formé du polyisoprène, de l'uréthane et de la silicone.

6. Appareil chirurgical (10, 20) selon l'une quelconque des revendications 1 à 3, dans lequel l'élément d'ancrage étanche (100, 200) est formé d'un matériau de type gel.

7. Appareil chirurgical (10, 20) selon l'une quelconque des revendications précédentes, dans lequel le au moins un orifice (108, 208) inclut au moins une dépouille (118) pour protéger contre les fuites.

8. Appareil chirurgical (10, 20) selon l'une quelconque des revendications précédentes, dans lequel l'appareil chirurgical (10, 20) peut être découpé.

9. Appareil chirurgical (10, 20) selon l'une quelconque des revendications précédentes, dans lequel l'élément d'ancrage étanche (100, 200) inclut une pluralité d'orifices (108, 208).

10. Appareil chirurgical (10, 20) selon la revendication 9, dans lequel la pluralité d'orifices (108, 208) est configurée de manière linéaire par rapport à l'axe principal du périmètre d'au moins l'une des extrémités distale et proximale.

11. Appareil chirurgical (10, 20) selon la revendication 9, dans lequel chaque orifice (108, 208) de la pluralité d'orifices (108, 208) est espacé de manière égale de ses orifices voisins.

12. Appareil chirurgical (10, 20) selon la revendication 9, dans lequel l'élément d'ancrage étanche (100, 200) inclut une pluralité de segments d'orifice de forme générale tubulaire, les segments d'orifice définissant des orifices respectifs.
